(19) **Europäisches Patentamt / European Patent Office / Office européen des brevets**

(11) **EP 4 104 826 A1**

(12) **EUROPEAN PATENT APPLICATION**
published in accordance with Art. 153(4) EPC

(43) Date of publication:
**21.12.2022 Bulletin 2022/51**

(21) Application number: **21753213.4**

(22) Date of filing: **09.02.2021**

(51) International Patent Classification (IPC):
**A61K 31/196** (2006.01)  **A61K 9/70** (2006.01)
**A61K 47/06** (2006.01)  **A61K 47/12** (2006.01)
**A61K 47/14** (2017.01)  **A61K 47/20** (2006.01)
**A61K 47/32** (2006.01)  **A61K 47/34** (2017.01)
**A61P 29/00** (2006.01)

(52) Cooperative Patent Classification (CPC):
**A61K 9/70; A61K 31/196; A61K 47/06;**
**A61K 47/12; A61K 47/14; A61K 47/20;**
**A61K 47/32; A61K 47/34; A61K 47/44;**
**A61K 47/46; A61P 29/00**

(86) International application number:
**PCT/JP2021/004819**

(87) International publication number:
**WO 2021/162004 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.02.2020 JP 2020021280**

(71) Applicant: **Hisamitsu Pharmaceutical Co., Inc.
Tosu-shi, Saga 841-0017 (JP)**

(72) Inventors:
- **SATO Hiroyuki
  Tokyo 100-6330 (JP)**
- **INAKURA Hiroshi
  Tokyo 100-6330 (JP)**

(74) Representative: **Grünecker Patent- und
Rechtsanwälte
PartG mbB
Leopoldstraße 4
80802 München (DE)**

(54) **DICLOFENAC SODIUM-CONTAINING ADHESIVE PATCH**

(57) The present invention provides a patch for relieving lumbago, the patch comprising a backing and an adhesive layer laminated on the backing, wherein the adhesive layer comprises an adhesive base and 0.70 to 1.50 mg of diclofenac sodium per cm$^2$ of application area, and the patch is used by applying the patch once a day to the chest region, the abdominal region, the dorsal region, the brachial region, or the femoral region of a human.

*Fig.3*

EP 4 104 826 A1

## Description

### Technical Field

The present invention relates to a diclofenac-containing patch.

### Background Art

A patch is a type of percutaneous absorption formulation manufactured by spreading out a plaster containing a medical agent on a backing. Application of the patch to the skin close to the affected region has an advantage of increasing the tissue concentration of the medical agent topically, while avoiding the first pass effect in the liver.

### Citation List

### Patent Literature

[Patent Literature 1] WO 2013/191128 A1

### Summary of Invention

### Technical Problem

Lumbago is a general term for symptoms mainly characterized by pain, tension, and the like in the lumbar region caused by (1) a motion factor (for example, a case where excessive load is applied dynamically or statically to the lumbar region), (2) an environmental factor (for example, vibration in the lumbar region, cold, falling on the floor or stairs), and (3) an individual factor (for example, differences in age, sex, physical constitution, and muscle strength, the presence of underlying diseases such as lumbar disc herniation and osteoporosis, and mental strain).

When a patch is applied for the purpose of treating lumbago, it is usually applied to the affected region, that is, lumbar region. However, the lumbar region is a location that is difficult for the patient to reach by their own hands, and it may be difficult for elderly patients, patients who live alone, or patients with physical disabilities to apply the patch to the lumbar region.

Up to now, patches containing medical agents at high concentrations have been investigated (for example, Patent Literature 1). However, unlike a case where they are applied near the affected region (topical administration), when they are applied to sites other than the affected region, they are affected by distribution to other tissues due to blood circulation, hepatic metabolism, and renal excretion, and therefore, it is unclear whether simply increasing the content of medical agents will achieve the desired effect of relieving lumbago. Thus, an object of the present invention is to provide a patch that is effective in relieving lumbago even when applied to a location other than the lumbar region.

### Solution to Problem

The present inventors have found that a patch in which an adhesive layer contains an adhesive base and diclofenac sodium and the content of diclofenac sodium is 0.70 to 1.50 mg per $cm^2$ of application area is effective in relieving lumbago even when applied to the non-lumbar region, and have thus completed the present invention.

A patch of the present invention is a patch for relieving lumbago, the adhesive patch comprising a backing and an adhesive layer laminated on the backing, wherein the adhesive layer contains an adhesive base and 0.70 to 1.50 mg of diclofenac sodium per $cm^2$ of application area, and the patch is used by applying the patch once a day to the chest region, the abdominal region, the dorsal region, the brachial region, or the femoral region of a human.

### Advantageous Effects of Invention

A patch according to the present invention is effective in relieving lumbago even when applied to a location other than the lumbar region.

### Brief Description of Drawings

[Fig. 1] Fig. 1 shows the overview of a test to evaluate the effect of relieving lumbago.

[Fig. 2] Fig. 2 is a graph showing the relationship between the lumbar region application rate and the change in VAS value.

[Fig. 3] Fig. 3 is a graph showing the change over time of the average VAS values in Group A and Group B.

**Description of Embodiments**

Hereinafter, the present invention will be described in detail.

One embodiment of the present invention is a patch for relieving lumbago, the patch comprising a backing and an adhesive layer laminated on the backing, wherein the adhesive layer contains an adhesive base and 0.70 to 1.50 mg of diclofenac sodium per $cm^2$ of application area, and the patch is used by applying the patch once a day to the chest region, the abdominal region, the dorsal region, the brachial region, or the femoral region of a human. In the patch, a peelable film is laminated on the other surface of the adhesive layer (the surface opposite to the surface on which the backing is laminated), if needed.

The lumbar region means, in anatomical sense, the dorsal region around the lumbar vertebrae. The spinal column is formed from about 30 vertebrae: cervical vertebrae (7 vertebrae), thoracic vertebrae (12 vertebrae), lumbar vertebrae (5 vertebrae), sacral vertebrae (5 vertebrae), and caudal vertebrae (3 to 6 vertebrae). Accordingly, in the present specification, the lumbar region refers to the part of the human posterior surface corresponding to the position where the 20th to 24th vertebrae, counting from the cervical side, exist, and the parts above and below this lumbar region are defined as the upper back region and the gluteal region, respectively. The chest region means the range from the cervical region to the diaphragm in the human anterior surface, and the abdominal region means the range from the diaphragm to the hip joint in the human anterior surface. The brachial region means the range from the shoulder joint to the elbow joint, and the femoral region means the range from the hip joint to the knee joint. The brachial region and the femoral region may be any of the anterior surface, posterior surface, and lateral surface of a human.

At first, the adhesive layer will be described. The adhesive layer is a site that is to be pressed onto the skin when applying the patch, and contains the adhesive base and diclofenac sodium.

Diclofenac sodium is a non-steroidal anti-inflammatory drug (NSAID) and a pain-relieving component. The patch of the present embodiment contains 0.70 to 1.50 mg of diclofenac sodium per $cm^2$ of application area. In addition, the patch of the present embodiment contains preferably 50 to 100 mg, more preferably 60 to 90 mg, and still more preferably 70 to 80 mg of diclofenac sodium per patch.

The adhesive base may contain at least one adhesive base selected from a rubber adhesive base, an acrylic adhesive base, and a silicone adhesive base. The rubber adhesive base is, for example, a polyisoprene, a polyisobutylene (PIB), a polybutadiene, a styrene-butadiene-styrene block copolymer, a styrene-isoprene-styrene (SIS) block copolymer, a styrene-butadiene rubber, a styrene-isoprene rubber, or a combination thereof. Among the above, from the points of enhancing the skin permeability of diclofenac sodium and further enhancing the adhesiveness of the patch, a SIS block copolymer, a PIB, or a combination thereof is preferred, and a mixture of a SIS block copolymer and a PIB is more preferred. The acrylic adhesive base is, for example, an adhesive base in which at least one of (meth)acrylic monomers, such as (meth)acrylic acid, 2-ethylhexyl (meth)acrylate, methyl (meth)acrylate, butyl (meth)acrylate, and hydroxyethyl (meth)acrylate, is polymerized or copolymerized. The silicone adhesive base is, for example, mainly composed of a silicone rubber, such as polydimethylsiloxane, polymethylvinylsiloxane, and polymethylphenylsiloxane.

In the case of rubber adhesive base, from the point of the adhesiveness of the patch, the content of the adhesive base may be 10 to 70 mass%, 20 to 50 mass%, 23 to 40 mass%, or 25 to 30 mass% with respect to the entire mass of the adhesive layer. In the case of acrylic adhesive base or silicone adhesive base, it is preferable that the content of the adhesive base be 50 to 90 mass% with respect to the entire mass of the adhesive layer. When the rubber adhesive base is a mixture of a SIS block copolymer and a PIB, it is preferable that the mass ratio of the SIS block copolymer and the PIB be 4:1 to 1:4, 3:1 to 1:1, or 3:1 to 2:1.

The adhesive layer may further contain DMSO for the purpose of, for example, dissolving diclofenac sodium and improving its skin permeability. It is preferable that the content of dimethyl sulfoxide (DMSO) be 1 to 20 mass%, 2 to 10 mass%, 3 to 10 mass%, or 5 to 9 mass% with respect to the entire mass of the adhesive layer.

From the point of improving the skin permeability of diclofenac sodium and from the point of preventing the precipitation of diclofenac sodium crystals, the ratio of diclofenac sodium and DMSO may be 1:0.3 to 1:4, and it is preferable that the ratio be 1:0.4 to 1:3, 1:0.6 to 1:3, or 1:0.72 to 1:3.

For the purpose of promoting percutaneous absorption of diclofenac sodium, preventing the precipitation of diclofenac sodium crystals over time, or other reasons, the adhesive layer may further contain an organic acid. Examples of the organic acid include: aliphatic carboxylic acids, such as aliphatic monocarboxylic acids (formic acid, acetic acid, propionic acid, butyric acid, isobutyric acid, valeric acid, caproic acid, enanthic acid, caprylic acid, nonanoic acid, capric acid, lauric acid, oleic acid, linoleic acid, linolenic acid, isostearic acid, sorbic acid, pyruvic acid, and others), aliphatic dicarboxylic acids (oxalic acid, malonic acid, succinic acid, glutaric acid, adipic acid, sebacic acid, maleic acid, fumaric acid, oxaloacetic acid, and others), and aliphatic tricarboxylic acids (aconitic acid, propanetricarboxylic acid, and others);

hydroxy acids (glycolic acid, lactic acid, tartronic acid, glyceric acid, hydroxybutyric acid, malic acid, tartaric acid, citric acid, isocitric acid, saccharic acid, gluconic acid, glucuronic acid, ascorbic acid, erythorbic acid, and others); aromatic carboxylic acids (benzoic acid, gallic acid, salicylic acid, acetylsalicylic acid, phthalic acid, and others); other organic acids (mesylic acid, besylic acid, and others); and salts thereof (for example, alkali metal salts such as sodium salts). One of these organic acids may be used singly, or two or more of them may be used in combination. Among these organic acids, from the points of promoting percutaneous absorption of diclofenac sodium and preventing the precipitation of diclofenac sodium crystals over time, in particular, citric acid, oleic acid, mesylic acid, or alkali metal salts thereof are preferred, and oleic acid is more preferred. From the points of improving the skin permeability of diclofenac sodium and preventing the precipitation of diclofenac sodium crystals over time, it is preferable that the content of the organic acid be 0.1 to 20 mass%, 0.5 to 10 mass%, 1 to 8 mass%, or 2 to 7 mass% with respect to the entire mass of the adhesive layer.

The adhesive layer may further contain other additives such as a tackifier, a plasticizer, a dissolving agent, a stabilizer, and a filler.

Examples of the tackifier include rosin and rosin derivatives such as rosin glycerol esters, hydrogenated rosins, hydrogenated rosin glycerol esters, and rosin pentaerythritol esters; alicyclic saturated hydrocarbon resins such as Arkon PI00 (trade name, manufactured by Arakawa Chemical Industries, Ltd.); aliphatic hydrocarbon resins such as Quintone B170 (trade name, manufactured by ZEON Corporation); terpene resins such as Clearon P-125 (trade name, manufactured by Yasuhara Chemical Co., Ltd.); and maleic acid resins. Among the above, hydrogenated rosin glycerol esters, alicyclic saturated hydrocarbon resins, aliphatic hydrocarbon resins, or terpene resins are preferred, and alicyclic hydrocarbon resins are more preferred. Two or more of these adhesiveness-imparting resins may be combined. When the adhesiveness-imparting resin is contained, the adhesive property of the adhesive layer can be improved and other physical properties can be stably maintained. It is preferable that the content of the adhesiveness-imparting resin be, for example, 5 to 60 mass%, 10 to 50 mass%, 30 to 45 mass%, or 35 to 40 mass% based on the entire mass of the adhesive layer. As the tackifier, the combination of an alicyclic saturated hydrocarbon resin and a hydrogenated rosin glycerol ester is more preferred. It is preferable that the mass ratio of the alicyclic saturated hydrocarbon resin and the hydrogenated rosin glycerol ester be 4:1 to 1:4, 4:1 to 2:3, or 3:1 to 2:1.

Examples of the plasticizer include: petroleum oils such as paraffinic process oils, naphthenic process oils, and aromatic process oils; squalane; squalene; vegetable oils such as olive oil, camellia oil, castor oil, tall oil, and peanut oil; silicone oil; dibasic acid esters such as dibutyl phthalate and dioctyl phthalate; liquid rubbers such as polybutenes and liquid isoprene rubbers; liquid fatty acid esters such as isopropyl myristate, hexyl laurate, diethyl sebacate, and diisopropyl sebacate; diethylene glycol; polyethylene glycol; glycol salicylate; propylene glycol; dipropylene glycol; triacetin; triethyl citrate; and crotamiton. Among the above, liquid paraffin, liquid polybutene, isopropyl myristate, diethyl sebacate, and hexyl laurate are preferred, liquid polybutene, isopropyl myristate, and liquid paraffin are more preferred, and liquid paraffin is particularly preferred. Two or more of these plasticizers may be combined. It is preferable that the content of the plasticizer be, for example, 7 to 70 mass%, 8 to 40 mass%, 10 to 20 mass%, or 12 to 15 mass% with respect to the entire mass of the adhesive layer.

Examples of the dissolving agent include liquid fatty acid esters (isopropyl myristate, hexyl laurate, diethyl sebacate, diisopropyl sebacate, and others), diethylene glycol, triethylene glycol, polyethylene glycol, propylene glycol, dipropylene glycol, triacetin, triethyl citrate, and crotamiton. Among the above, diethylene glycol, triethylene glycol, polyethylene glycol, propylene glycol, and dipropylene glycol are preferred, and polyethylene glycol is more preferred. When the dissolving agent is polyethylene glycol, the number average molecular weight of polyethylene glycol may be 200 to 20000, and is preferably 400 to 6000, and more preferably 1000 to 6000. Two or more of these dissolving agents may be combined. The content of the dissolving agent is, for example, 0.1 to 10 mass% or 0.5 to 5 mass% based on the entire mass of the adhesive layer.

Examples of the stabilizer include fatty acid metal salts (magnesium stearate and others), antioxidants (tocopherol derivatives, ascorbic acid derivatives, erythorbic acid derivatives, nordihydroguaiaretic acid, gallic acid derivatives, dibutylhydroxytoluene (BHT), butylhydroxyanisole, 2-mercaptobenzimidazole, and others), and ultraviolet absorbers (imidazole derivatives, benzotriazole derivatives, p-aminobenzoic acid derivatives, anthranilic acid derivatives, salicylic acid derivatives, cinnamic acid derivatives, benzophenone derivatives, coumaric acid derivatives, camphor derivatives, and others). Two or more of these stabilizers may be combined. The content of the stabilizer is preferably 0 to 5 mass% and more preferably 0 to 2 mass% based on the entire mass of the adhesive layer. When the adhesive layer contains the stabilizer, the stability of diclofenac sodium can be further improved.

Examples of the filler include metal oxides (zinc oxide, titanium oxide, and others), metal salts (calcium carbonate, magnesium carbonate, zinc stearate, and others), silicate compounds (kaolin, talc, bentonite, aerosil, hydrous silica, aluminum silicate, magnesium silicate, magnesium aluminometasilicate, and others), and metal hydroxides (aluminum hydroxide and others). Two or more of these fillers may be combined. The content of the filler is preferably 0 to 10 mass% and more preferably 0 to 5 mass% based on the entire mass of the adhesive layer.

The adhesive layer may be a single layer consisting of one composition or a multilayer prepared by laminating multiple layers of different compositions. From the point of allowing the patch to appropriately adhere to the skin, the entire plaster mass of the adhesive layer is preferably 10 to 1000 g/m$^2$, more preferably 30 to 300 g/m$^2$, and still more preferably 150 to 250 g/m$^2$.

Next, the backing will be described. The backing retains the adhesive layer. It is preferable that the backing be a monolayer or laminate of fibers made into the form of a fabric (woven fabric, non-woven fabric, or knitted fabric), or a non-porous or porous film (sheet). It is preferable that the material of the backing be one or more materials selected from polyesters (polyethylene terephthalate (PET), polyethylene isophthalate, polypropylene terephthalate, polypropylene isophthalate, polybutylene terephthalate, polyethylene naphthalate, or the like), polyolefins (polymers or copolymers of vinyl monomers such as ethylene, propylene, vinyl acetate, or acrylonitrile), polyamides (nylon, silk, or the like), polyurethanes (PU), or celluloses (cotton, linen, or the like). The fabric (woven fabric, non-woven fabric, or knitted fabric) may be coated with a rubber composition. The rubber composition contains a rubber adhesive base. The rubber adhesive base is, for example, a polyisoprene, a PIB, a polybutadiene, a styrene-butadiene-styrene block copolymer, a SIS block copolymer, a styrene-butadiene rubber, a styrene-isoprene rubber, or a combination thereof. The rubber composition may contain a tackifier. The tackifier is, for example, an alicyclic saturated hydrocarbon resin, a hydrogenated rosin ester, a terpene resin, or a combination thereof. In addition, the rubber composition may further contain additives such as a plasticizer and a filler. The thickness of the backing is, for example, 0.1 to 2 mm. The basis weight of the backing is, for example, 30 to 200 g/m$^2$. In the present specification, the thickness and the basis weight of the backing are measured in accordance with the standard of JIS L 1906:2000.

It is preferable that the water vapor transmission rate of the backing be 1000 g/m$^2$·24 hr or more. When using a backing with such a high water vapor transmission rate, DMSO is gradually volatilized from the patch applied to the skin, which improves the adhesiveness of the patch and prevents it from being peeled off even if the patch is applied for a long period of time. The upper limit value of water vapor transmission rate may be 20000 g/m$^2$·24 hr. When the water vapor transmission rate of the backing is in such a range, DMSO is more easily volatilized from the adhesive layer, which is more effective in improving the adhesiveness of the patch. Note that the water vapor transmission rate of the backing means the water vapor transmission rate at 40°C as defined in the standard of JIS Z 0208:1976 (Testing Methods for Determination of the Water Vapor Transmission Rate of Moisture-Proof Packaging Materials (Dish method)).

When the backing is in the form of a fabric, it is preferable that the 50% moduluses (measured in accordance with the standard of JIS L 1018:1999 or JIS L 1096:2010) in both the longitudinal direction (material flow direction) and the transverse direction (material width direction) of the backing be 1 N/50 mm to 12 N/50 mm. When the 50% moduluses are 12 N/50 mm or less, the stress to which the patch is subjected due to skin expansion and contraction is smaller, resulting in better attachment to the skin.

When the backing is a film, it is preferable that the material be highly water vapor transmissible (highly DMSO transmissible), such as polyurethane. A film consisting of polyurethane is excellent in elasticity, and is thus preferred from the point of enhancing the attachment and expansion and contraction followability of the patch to the skin.

It is preferable that the backing be, for example, a non-woven fabric or film consisting of polyurethane, a knitted fabric consisting of polyethylene terephthalate, a polyester fabric coated with a rubber composition, or a combination thereof. More specifically, it is preferable that the backing be a laminate of a film consisting of polyurethane and a non-woven fabric consisting of polyurethane fibers, a non-woven fabric consisting of PET fibers, or a polyester fabric coated with a rubber composition.

In order to ensure sufficient adhesive force and to ensure a sufficient amount of diclofenac sodium transmission through the skin, the application area of the patch is preferably 30 to 300 cm$^2$, and more preferably 50 to 150 cm$^2$. The content of diclofenac sodium according to the present embodiment is 0.70 to 1.50 mg per cm$^2$ of application area, and is thus 35 to 225 mg per patch. The preferred content of diclofenac sodium is 50 to 100 mg per patch.

The patch can be produced by, for example, the following method, but the production method is not limited to this and any known method can be used. At first, each component constituting the adhesive layer is mixed at a predetermined proportion to obtain a uniform dissolved product. Next, the dissolved product is spread over a peelable film (release film, release liner) at a predetermined thickness to form the adhesive layer. Then, the backing is pressed onto the adhesive layer so that the adhesive layer is sandwiched between the release liner and the backing. Finally, by cutting into the desired shape, the patch can be obtained. In this case, the release liner is removed at the time of application of the patch. Alternatively, in the same manner, the dissolved material may be spread over the backing at a predetermined thickness to form the adhesive layer, and the backing may then be pressed onto a peelable film (release film, release liner).

For human adults, at least one patch is applied to the chest region, the abdominal region, the dorsal region, the brachial region, the femoral region, or other regions once a day, and is changed every day (approximately 24 hours). When the effect of relieving lumbago is not achieved sufficiently, two or three patches may be applied once a day. In order to avoid skin irritation, it is desirable to change the site of application each time.

By applying the patch of the present embodiment once a day to the chest region, the abdominal region, the dorsal region, the brachial region, or the femoral region of a human (for example, repeated administration for 4 weeks), the average plasma concentration of diclofenac sodium can be 20 to 90 ng/mL. Even when applied to the non-lumbar region, if the average plasma concentration is maintained within the range described above, lumbago can be relieved more reliably.

The present embodiment has the following aspects, as well.

[1] A patch for relieving lumbago, the patch comprising a backing and an adhesive layer laminated on the backing, wherein the adhesive layer comprises an adhesive base and 0.70 to 1.50 mg of diclofenac sodium per $cm^2$ of application area, and the patch is used by applying the patch once a day to the chest region, the abdominal region, the dorsal region, the brachial region, or the femoral region of a human.

[2] The patch according to [1], wherein the adhesive base comprises at least one selected from the group consisting of a styrene-isoprene-styrene block copolymer and a polyisobutylene.

[3] The patch according to [2], wherein the adhesive base comprises the styrene-isoprene-styrene block copolymer and the polyisobutylene.

[4] The patch according to any of [1] to [3], wherein the adhesive layer further comprises dimethyl sulfoxide.

[5] The patch according to any of [1] to [4], wherein the adhesive layer further comprises an organic acid.

[6] The patch according to [5], wherein the organic acid is oleic acid.

[7] The patch according to any of [1] to [6], wherein the adhesive layer further comprises a tackifier.

[8] The patch according to [7], wherein the tackifier is an alicyclic saturated hydrocarbon resin and a hydrogenated rosin glycerol ester.

[9] The patch according to any of [1] to [8], wherein the adhesive layer comprises a plasticizer.

[10] The patch according to [9], wherein the plasticizer is liquid paraffin.

[11] The patch according to any of [1] to [10], wherein the content of diclofenac sodium is 50 to 100 mg.

[12] The patch according to any of [1] to [11], wherein the number of patches per application is 1 to 3.

[13] The patch according to any of [1] to [12], wherein the backing is a polyethylene terephthalate knitted fabric having a water vapor transmission rate of 1000 $g/m^2 \cdot 24$ hr or more.

[14] The patch according to any of [1] to [13], wherein the application area is 50 to 150 $cm^2$.

[15] A method for relieving lumbago of a human, the method comprising applying a patch to the human, the patch comprising a backing and an adhesive layer laminated on the backing; the adhesive layer comprising an adhesive base and 0.70 to 1.50 mg of diclofenac sodium per $cm^2$ of application area; and wherein the patch is applied once a day to the chest region, the abdominal region, the dorsal region, the brachial region, or the femoral region of the human.

[16] The method according to [15], the patch comprising at least one selected from the group consisting of a styrene-isoprene-styrene block copolymer and a polyisobutylene as the adhesive base.

[17] The method according to [16], the patch comprising the styrene-isoprene-styrene block copolymer and the polyisobutylene as the adhesive base.

[18] The method according to any of [15] to [17], the patch further comprising dimethyl sulfoxide in the adhesive layer.

[19] The method according to any of [15] to [18], the patch further comprising an organic acid in the adhesive layer.

[20] The method according to [19], the patch comprising oleic acid as the organic acid.

[21] The method according to any of [15] to [20], the patch further comprising a tackifier in the adhesive layer.

[22] The method according to [21], the patch comprising an alicyclic saturated hydrocarbon resin and a hydrogenated rosin glycerol ester as the tackifier.

[23] The method according to any of [15] to [22], the patch further comprising a plasticizer in the adhesive layer.

[24] The method according to [23], the patch comprising liquid paraffin as the plasticizer.

[25] The method according to any of [15] to [24], wherein the content of diclofenac sodium is 50 to 100 mg.

[26] The method according to any of [15] to [25], wherein the number of patches per application is 1 to 3.

[27] The method according to any of [15] to [26], wherein the patch uses a polyethylene terephthalate knitted fabric having a water vapor transmission rate of 1000 $g/m^2 \cdot 24$ hr or more as the backing.

[28] The method according to any of [15] to [27], wherein the patch is applied to an area of 50 to 150 $cm^2$.

(Preparation of Patch)

A patch containing diclofenac sodium was prepared by the following method. Each component shown in Table 1 was mixed and spread over a release liner (PET film that had been subjected to release treatment) so that the plaster mass was 214 $g/m^2$. A backing was laminated on the spread plaster solution (adhesive layer), and the laminate was cut into rectangles with a size of 7 cm × 10 cm per patch, thereby obtaining patches (application area: 70 $cm^2$). A PET knitted fabric having a water vapor transmission rate of 5667 $g/m^2 \cdot 24$ hr was used as the backing. The content of diclofenac sodium per patch is 75 mg.

[Table 1]

| Component | Example 1 |
|---|---|
| Diclofenac sodium | 5 |
| Rubber adhesive base (SIS:PIB = 19:8) | 27 |
| Adhesiveness-imparting resin (alicyclic saturated hydrocarbon resin:hydrogenated rosin glycerol ester = 27:10) | 37 |
| Liquid paraffin | 13 |
| Oleic acid | 5 |
| DMSO | 7 |
| Other components | 6 |
| Total | 100 |

(Preparation of Placebo Patch)

A placebo patch with the same composition except that it did not contain diclofenac sodium was prepared in the same manner as described above.

(Evaluation of Effect of Relieving Lumbago)

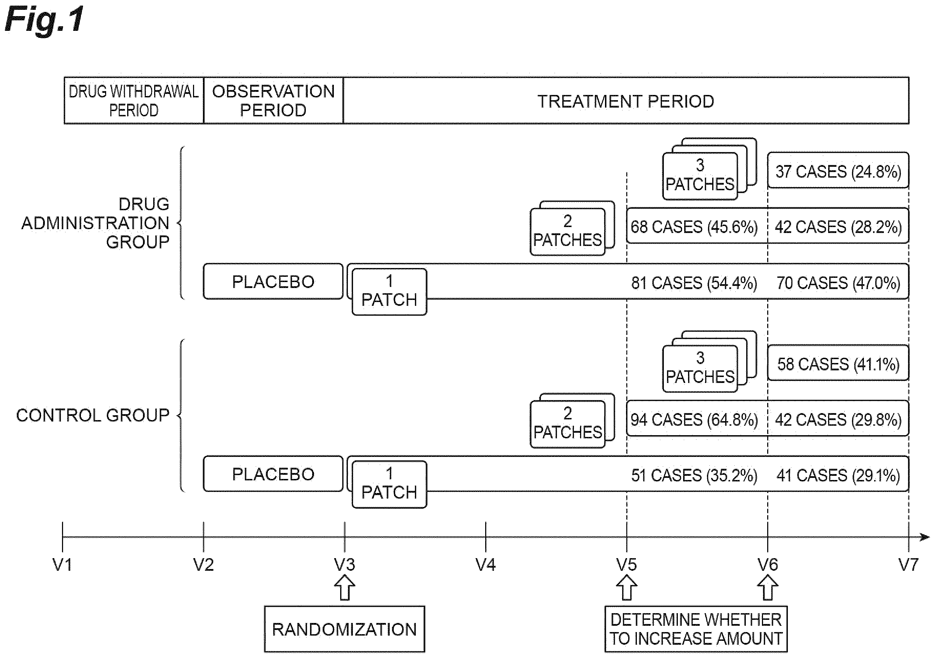

Figure 1 shows the overview of an evaluation test. In Figure 1, the interval between each of selection date or transition judgment dates (VI, V2, V3, and others) is 7 days.

At first, on patient selection date V1, patients who met all of the following selection criteria were given a drug withdrawal period of one week (including selection date V1).

<Selection Criteria>

(1-1) The patient had been clinically diagnosed with lumbago for at least 3 months prior to obtaining consent.

(1-2) The patient has been continuously treated with a NSAID or acetaminophen for lumbago for at least 4 weeks prior to obtaining consent and its usage and dosage have not been changed within 4 weeks prior to obtaining consent.

The VAS (visual analogue scale) values for the 3 days immediately before transition judgment date V2 (not including transition judgment date V2) were evaluated, and patients who met all of the following transition criteria on transition judgment date V2 were advanced to an additional 1-week observation period (including transition judgment date V2). During the observation period, one placebo patch was applied per day in a single-blind manner and changed every day. The patient himself/herself selected the site of application from the chest region, the lumbar region, the abdominal region, the dorsal region, the brachial region, the femoral region, and other regions on each occasion, and recorded it.

<Transition Criteria to Observation Period>

(2-1) The average value of the VAS values for the 3 days immediately before transition judgment date V2 (not including transition judgment date V2) is 40 mm or more.

(2-2) Compared to the VAS value before the drug withdrawal period, the average VAS value for the 3 days immediately before transition judgment date V2 (not including transition judgment date V2) is increased by 10 mm or more.

(2-3) Compared to before the drug withdrawal period, the degree of satisfaction for treatment by the patient is not improved at transition judgment date V2.

Patients who met all of the following selection criteria at transition judgment date V3 were advanced to a treatment period.

<Transition Criteria to Treatment Period>

(3-1) The average value of the VAS values for the 3 days immediately before transition judgment date V3 (not including transition judgment date V3) is 40 mm or more.

(3-2) Each VAS value for the 3 days immediately before transition judgment date V3 (not including transition judgment date V3) is within ±15 mm of the average value described above.

(3-3) The average value of the VAS values for the 3 days immediately before transition judgment date V3 (not including transition judgment date V3) is not decreased by 20 mm or more compared to the average VAS value for the 3 days immediately before transition judgment date V2.

(3-4) At transition judgment date V2, the AST (aspartate aminotransferase) value is 3 times the reference value upper limit (40 U/L) or less, the ALT (alanine aminotransferase) value is 3 times the reference value upper limit (45 U/L) or less, and the serum creatinine value is 1.5 times the reference value upper limit (1.04 mg/dL for males and 0.79 mg/dL for females) or less.

Patients were divided into a drug administration group and a control group, and one patch containing diclofenac sodium and one placebo patch were applied once a day to the drug administration group and once a day to the control group, respectively, in a double-blind manner, and were changed every day. The patient himself/herself selected the site of application from the chest region, the lumbar region, the abdominal region, the dorsal region, the brachial region, the femoral region, and other regions on each occasion. At and after transition judgment date V3, the VAS value was evaluated every day. If, at each of judgment dates V5 and V6, it was determined that "the average VAS value for the three days immediately before that date is not improved from the VAS value at the time of judgment date V3 by 15 mm or more" and there was no problem for safety reasons, the number of patches to be applied was increased by one. That is, for patients who were determined to have not improved from the average value of the VAS values for the 3 days immediately before transition judgment date V3 (not including transition judgment date V3) by 15 mm or more on both judgment dates V5 and V6, three patches were applied at and after judgment date V6.

Based on the obtained data, the effect of relieving lumbago was evaluated.

(1) Comparison by Lumbar Region Application Rate

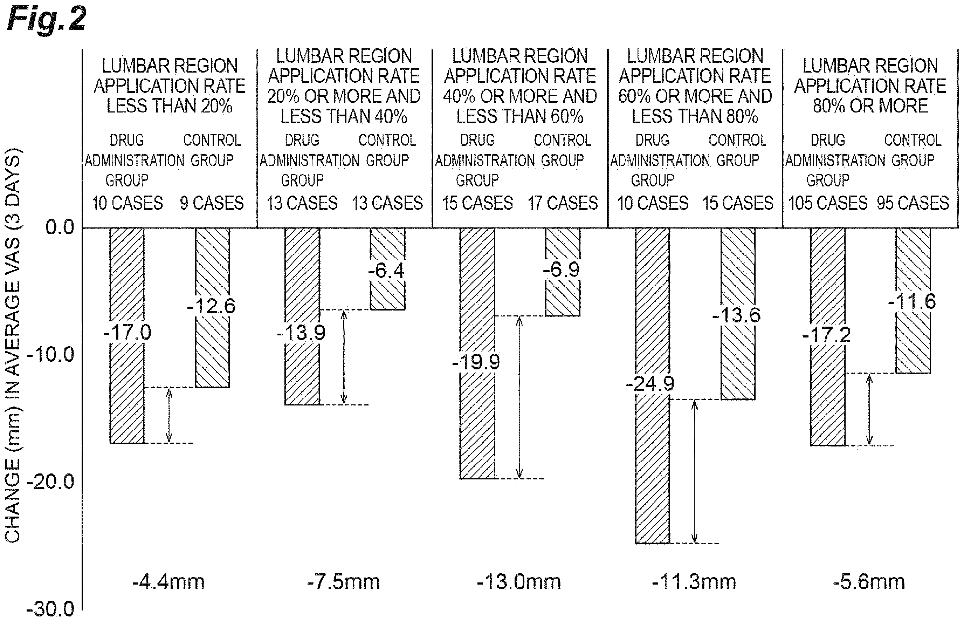

The lumbar region application rate was calculated from the following expression, and the results of comparing the lumbar region application rate with the change in the average value of the VAS values for the 3 days immediately before judgment date V5 (not including judgment date V5) are shown in Figure 2. Regardless of the level of the lumbar region application rate, the decrease in VAS value was greater in the drug administration group as compared to the control group.

$$\text{Lumbar region application rate (\%)} = \text{Number of days for}$$

$$\text{application to lumbar region / Number of days from transition judgment}$$

$$\text{date V3 to the day before judgment date V5} \times 100$$

(2) Comparison between Application Group to Lumbar Region and Application Group to Non-Lumbar Region

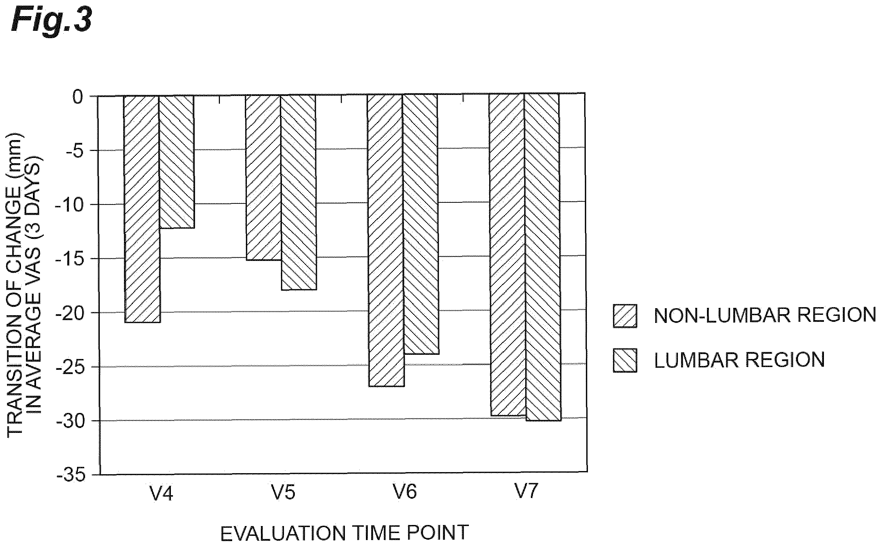

From the obtained data, the data were extracted and analyzed for each of the following groups: a group of application only to the lumbar region (Group A), and a group of application to a site other than the lumbar region, but not to the lumbar region (Group B). The average values of the average VAS values for the 3 days immediately before each of judgment date V4, V5, V6, and V7 in Group A and Group B are shown in Table 2 and Figure 3. In Table 2, the numbers in parentheses mean the number of patients counted according to the site of application during the 3 days immediately before the judgment date.

[Table 2]

| Group | Site of application | V4 | V5 | V6 | V7 |
|---|---|---|---|---|---|
| A | Lumbar region | -12.23 (101) | -18.06 (98) | -24.09 (80) | -30.27 (69) |

(continued)

| Group | Site of application | V4 | V5 | V6 | V7 |
|---|---|---|---|---|---|
| B | Non-lumbar region | -20.90 (17) | -15.31 (17) | -26.98 (15) | -29.77 (16) |

The change in the average value of the VAS values in Group B did not significantly differ from the change in the average value of the VAS values in Group A. It was found that the patch of the present invention, even when applied to the non-lumbar region (chest region, abdominal region, dorsal region, brachial region, or femoral region), exhibited the effect of relieving lumbago to the same extent as when applied to the lumbar region (affected region).

(3) Pharmacokinetics at Single Administration

Two patches (application area: 70 cm$^2$) prepared according to the description in Table 1 were applied to the dorsal region of 14 healthy adult males, and peeled off after 24 hours. Blood samples were taken from immediately before to 36 hours after the start of application, and the blood drug concentrations were measured. From the obtained blood drug concentration at each time point, the maximum blood drug concentration ($C_{max}$) and its time ($T_{max}$) were determined. In addition, a graph was created with time on the abscissa and blood drug concentration on the ordinate, and the area under the curve (AUC) and the half-life of the drug were determined.

The results are shown in Table 3.

[Table 3]

| | $C_{max}$ [ng/mL] | $t_{max}$ [hr] | $AUC_{0-t}$ [ng · hr/mL] | $AUC_{0-24}$ [ng · hr/mL] | $AUC_{0-\infty}$ [ng · hr/mL] | $t_{1/2}$ [hr] |
|---|---|---|---|---|---|---|
| Number of cases | 14 | 14 | 14 | 14 | 14 | 14 |
| Average value | 55.7 | 13.6 | 1030 | 915 | 1030 | 2.85 |
| Standard deviation | 18.9 | 3.9 | 388 | 367 | 389 | 0.277 |

(4) Pharmacokinetics at Repeated Administration

One patch (application area: 70 cm$^2$) prepared according to the description in Table 1 was applied to the dorsal region of 14 healthy adult males, peeled off every 24 hours, and replaced with a new patch. This operation was repeated 14 times, and the patch was peeled off on day 15. Blood samples were taken from immediately before to 360 hours after the start of application, and the blood drug concentrations were measured. From the obtained blood drug concentration at each time point, the maximum blood drug concentration ($C_{max}$) and its time ($T_{max}$) were determined. In addition, a graph was created with time on the abscissa and blood drug concentration on the ordinate, and the area under the curve (AUC) and the half-life of the drug were determined.

The results are shown in Table 4.

[Table 4]

| | $C_{max}$ [ng/mL] | | | $t_{max}$ [hr] | | |
|---|---|---|---|---|---|---|
| | 1st time | 7th time | 14th time | 1st time | 7th time | 14th time |
| Number of cases | 14 | 13 | 13 | 14 | 13 | 13 |
| Average value | 22.9 | 44.1 | 64.0 | 12.9 | 10 | 6.2 |
| Standard deviation | 7.07 | 10.0 | 21.4 | 4.1 | 4.2 | 2.5 |
| | $AUC_{0-24}$ [ng · hr/mL] | | | $t_{1/2}$ [hr] | | |
| | 1st time | 7th time | 14th time | After final peeling | | |
| Number of cases | 14 | 13 | 13 | 13 | | |
| Average value | 372 | 813 | 1070 | 2.86 | | |
| Standard deviation | 126 | 169 | 299 | 1.44 | | |

**Claims**

1.  A patch for relieving lumbago, the patch comprising a backing and an adhesive layer laminated on the backing, wherein

    the adhesive layer comprises an adhesive base and 0.70 to 1.50 mg of diclofenac sodium per $cm^2$ of application area, and
    the patch is used by applying the patch once a day to a chest region, an abdominal region, a dorsal region, a brachial region, or a femoral region of a human.

2.  The patch according to claim 1, wherein the adhesive base comprises at least one selected from the group consisting of a styrene-isoprene-styrene block copolymer and a polyisobutylene.

3.  The patch according to claim 2, wherein the adhesive base comprises the styrene-isoprene-styrene block copolymer and the polyisobutylene.

4.  The patch according to any one of claims 1 to 3, wherein the adhesive layer further comprises dimethyl sulfoxide.

5.  The patch according to any one of claims 1 to 4, wherein the adhesive layer comprises an organic acid.

6.  The patch according to claim 5, wherein the organic acid is oleic acid.

7.  The patch according to any one of claims 1 to 6, wherein the adhesive layer further comprises a tackifier.

8.  The patch according to claim 7, wherein the tackifier is an alicyclic saturated hydrocarbon resin and a hydrogenated rosin glycerol ester.

9.  The patch according to any one of claims 1 to 8, wherein the adhesive layer comprises a plasticizer.

10. The patch according to claim 9, wherein the plasticizer is liquid paraffin.

11. The patch according to any one of claims 1 to 10, wherein a content of diclofenac sodium is 50 to 100 mg.

12. The patch according to any one of claims 1 to 11, wherein the number of patches per application is 1 to 3.

13. The patch according to any one of claims 1 to 12, wherein the backing is a polyethylene terephthalate knitted fabric having a water vapor transmission rate of 1000 $g/m^2 \cdot 24$ hr or more.

14. The patch according to any one of claims 1 to 13, wherein an application area is 50 to 150 $cm^2$.

# Fig.1

| DRUG WITHDRAWAL PERIOD | OBSERVATION PERIOD | TREATMENT PERIOD |

DRUG ADMINISTRATION GROUP

3 PATCHES — 37 CASES (24.8%)

2 PATCHES — 68 CASES (45.6%) | 42 CASES (28.2%)

PLACEBO | 1 PATCH — 81 CASES (54.4%) | 70 CASES (47.0%)

CONTROL GROUP

3 PATCHES — 58 CASES (41.1%)

2 PATCHES — 94 CASES (64.8%) | 42 CASES (29.8%)

PLACEBO | 1 PATCH — 51 CASES (35.2%) | 41 CASES (29.1%)

V1    V2    V3    V4    V5    V6    V7

RANDOMIZATION

DETERMINE WHETHER TO INCREASE AMOUNT

EP 4 104 826 A1

**Fig.2**

Fig.3

EP 4 104 826 A1

## INTERNATIONAL SEARCH REPORT

International application No.

PCT/JP2021/004819

### A. CLASSIFICATION OF SUBJECT MATTER

A61K 31/196(2006.01)i; A61K 9/70(2006.01)i; A61K 47/06(2006.01)i; A61K 47/12(2006.01)i; A61K 47/14(2006.01)i; A61K 47/20(2006.01)i; A61K 47/32(2006.01)i; A61K 47/34(2017.01)i; A61P 29/00(2006.01)i
FI:     A61K31/196; A61K9/70 401; A61K47/06; A61K47/12; A61K47/14; A61K47/20; A61K47/32; A61K47/34; A61P29/00

According to International Patent Classification (IPC) or to both national classification and IPC

### B. FIELDS SEARCHED

Minimum documentation searched (classification system followed by classification symbols)
A61K31/196; A61K9/70; A61K47/06; A61K47/12; A61K47/14; A61K47/20; A61K47/32; A61K47/34; A61P29/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| Y<br>A | WO 2013/191128 A1 (HISAMITSU PHARMACEUTICAL CO., INC.) 27 December 2013 (2013-12-27) claims 1, 3, paragraphs [0029], [0034], [0040], [0066], [0074], examples | 1, 4–14<br>2–3 |
| Y<br>A | WO 2018/141661 A1 (GRUNENTHAL GMBH) 09 August 2018 (2018-08-09) paragraphs [0066], [0074], [0111] | 1, 4–14<br>2–3 |
| Y | WO 2018/124089 A1 (HISAMITSU PHARMACEUTICAL CO., INC.) 05 July 2018 (2018-07-05) claim 3 | 13 |
| Y | WO 2010/137699 A1 (INOAC GIJUTSU KENKYUSHO KK) 02 December 2010 (2010-12-02) paragraphs [0002], [0013], [0014] | 13 |

☐ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 March 2021 (16.03.2021) | 30 March 2021 (30.03.2021) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**
Information on patent family members

International application No.

PCT/JP2021/004819

| Patent Documents referred in the Report | Publication Date | Patent Family | Publication Date |
|---|---|---|---|
| WO 2013/191128 A1 | 27 Dec. 2013 | US 2015/0202171 A1 claims 1, 3, paragraphs [0038], [0083], [0077], [0041], [0043], [0049], examples EP 2865378 A1 CN 104379139 A KR 10-2015-0023758 A | |
| WO 2018/141661 A1 | 09 Aug. 2018 | JP 2020-505416 A US 2019/0388360 A1 | |
| WO 2018/124089 A1 | 05 Jul. 2018 | US 2019/0350874 A1 claim 3 EP 3563840 A1 KR 10-2019-0085972 A CN 110114064 A | |
| WO 2010/137669 A1 | 02 Dec. 2010 | US 2012/0071808 A1 paragraphs [0002], [0027], [0028] EP 2436380 A1 CN 102448449 A KR 10-2012-0035167 A | |

Form PCT/ISA/210 (patent family annex) (January 2015)

**EP 4 104 826 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013191128 A1